# EUROPEAN PATENT APPLICATION

(11) **EP 1 288 649 A1**
(43) Date of publication of application: **05.03.2003**
(21) Application number: 01930005.2
(22) Date of filing: 09.05.2001
(51) Int. Cl.: G01N 21/27, G01N 21/75, G01N 33/44

(54) **METHOD AND DEVICE FOR DETECTING END POINT OF CURING OF RESIN, ASSEMBLY, APPARATUS AND METHOD FOR PRODUCING ASSEMBLY**

(30) Priority: 09.05.2000 JP 2000136213; 09.05.2000 JP 2000136228
(71) Applicant: HAMAMATSU PHOTONICS K. K., Hamamatsu-shi, Shizuoka 435-8558 (JP)
(72) Inventor: Kawai, Kazuhito, Hamamatsu-shi, Shizuoka 435-8558 (JP); Uchiyama, Naoki, Hamamatsu-shi, Shizuoka 435-8558 (JP); Matui, Ryotaro, Hamamatsu-shi, Shizuoka 435-8558 (JP); Ito, Shinobu, Hamamatsu-shi, Shizuoka 435-8558 (JP)
(74) Representative: Musker, David Charles
(86) International application number: JP0103874
(87) International publication number: WO01086261

(57) **Abstract**

Determined is a correlation value R (=I(t)/I(0)) between an intensity I(0) of monitor light at least at one specific wavelength which is detected before cure of resin RSN and an intensity I (t) of the monitor light at the specific wavelength which is thereafter detected, and the cure of the resin RSN is judged to be completed when a temporal change of the correlation value R comes to a stable state. In assembly manufacturing, light-cure resin RSN is interposed between at least two members M1 and M2. After the above described cure end point is detected, application of resin curing light to the resin RSN is stopped, and the assembly is transferred.

## Description

### Technical Field

The present invention relates to cure end point detection method and device for resin, specifically for light-cure resin. The present invention also relates to an assembly in which light-cure resin is used and manufacturing apparatus and method of the same.

### Background Art

Conventional cure end point detection methods and devices are described in Japanese Unexamined Patent Publication No. Hei5(1993)-45288, Japanese Unexamined Patent Publication No. Hei6(1994)-242005, and Japanese Unexamined Patent Publication No. Sho62 (1987)-103540. In these specifying methods, when monitor light transmittance of resin at a specific wavelength becomes a predetermined value, cure of the resin is judged to be completed.

### Disclosure of the Invention

However, it is impossible to perform an accurate cure end point judgment by such a method. In a case where a curing time exceeds appropriate end time, discoloration or deterioration of resin occurs. On the other hand, in a case where the curing time does not reach the appropriate end time, a defective product is manufactured as a matter of course. At a current mass production site to which the above described conventional method is applied, it is an actual situation that a worker picks at resin with a stick and the like to check cure status of the resin. The present invention has been made in view of the above described problem. It is an object of the present invention to provide resin cure end point detection method and device in which an accurate cure end point judgment can be performed.

In order to solve the above describedproblem, provided is a resin cure end point detection method according to the present invention, in which monitor light is applied to resin, any one of the monitor light reflected by the resin and the monitor light transmitted through the resin is detected, and a cure end point of the resin is detected, wherein, based on an intensity of the monitor light at least at one specific wavelength which is detected before cure of the resin and based on an intensity of the monitor light at the specific wavelength which is thereafter detected, the cure of the resin is judged to be completed.

When the intensity of the monitor light at least at the one specific wavelength which is detected before the cure of the resin and the intensity of the monitor light at the specific wavelength which is thereafter detected are used, an accurate cure end point judgment can be performed because these intensities are deeply related to the cure end point.

The monitor light before the cure is preferably detected by applying the monitor light to the resin before curing the resin.

As described above, the intensities before and after the cure are deeply related to the cure end point. Accordingly, in a method in which monitor light is applied to resin, any one of the monitor light reflected by the resin and the monitor light transmitted through the resin is detected, and a cure end point of the resin is detected, by determining a correlation value between an intensity of the monitor light at least at one specific wavelength which is detected before cure of the resin and an intensity of the monitor light at the specific wavelength which is thereafter detected, the cure of the resin can be judged to be completed when a temporal change of the correlation value comes to a stable state.

Use of the present invention permits a more accurate cure end point judgment. Note that "before curing resin" includes curing-start time.

The correlation value is preferably a ratio at the specific wavelength between the intensities before and after the cure of the resin. Moreover, in order to confirm the stable state, it is preferred that a time derivative of the correlation value is determined, and that the cure of the resin is judged to be completed when the determined time derivative becomes almost zero. Note that, where the correlation value is R, "almost zero" means that the time derivative (ΔR per minute) of the correlation value is in a range between -3% of R and +3% of R inclusive.

As a method of curing the resin, various methods are conceivable. If a device performing the relevant method further includes a resin curing light source which emits light with a wavelength for curing the resin to the resin, the cure can be started by the light. In this case, if a light source for emitting the monitor light is identical to the resin curing light source, a device configuration can be simplified.

A resin cure end point detection device performing the above described method has a feature of including a light source for emitting the monitor light, a light receiver for detecting the monitor light, and judging means for performing the judgment.

Amethod of manufacturing an assembly using light-cure resin has a feature of comprising the steps of: interposing light-cure resin between members of an assembly which includes at least two members, transferring the assembly to a position to which resin curing light for curing the resin is applied, and applying the resin curing light to the resin; and applying monitor light to the resin before applying the resin curing light, detecting any one of the monitor light reflected by the resin and the monitor light transmitted through the resin, detecting a cure end point of the resin based on a result of the foregoing detecting, stopping application of the resin curing light to the resin, and transferring the assembly from the position. Note that, in the former step, the interposing of the resin and the transferring of the assembly can be performed in random order. "Transferring an assembly" is assumed to mean that an assembly is resultingly transferred to the above described position.

According to the present method, the monitor light is applied to the resin before the resin curing light is applied, and the monitor light which is reflected by the resin or which is transmitted through the resin is detected. Based on a result of this detection, the cure end point of the resin is detected. Thereafter, application of the resin curing light to the resin is stopped, and the assembly is transferred from the curing light applying position. Accordingly, it is possible to manufacture an assembly efficiently. An assembly in which resin is cured according to the present manufacturing method can reduce manufacturing cost thereof.

As such an assembly, there are enumerated an optical pickup device and the like, which are described in Japanese Unexamined Patent Publication No. Hei10(1998)-39120, Japanese Unexamined Patent Publication No. Hei8(1996)-329508, Japanese Unexamined Patent Publication No. Hei9(1997)-35312, Japanese Unexamined Patent Publication No.Hei4(1992)-344792, and Japanese Unexamined Patent Publication No. Hei5(1993)-45288.

A manufacturing apparatus for performing a manufacturing method as described above has a feature of comprising a resin curing light source which applies resin curing light for curing light-cure resin to the resin; a cure status monitor light source for applying monitor light to the resin; a light receiver for detecting any one of the monitor light reflected by the resin and the monitor light transmitted through the resin; stopping means for stopping application of the resin curing light to the resin; and a controller for detecting a cure end point of the resin based on an output of the light receiver and for controlling the stopping means to stop the application of the resin curing light to the resin. Although it is possible to use as the stopping means a power supply switch of the resin curing light source, the stopping means is preferably a shutter interposed in a light path between the resin curing light source and the resin from a viewpoint of output stability.

### Brief Description of the Drawings

Fig. 1 is a block diagram of a resin cure end point detection device.
Fig. 2 is a graph showing a relationship between a light intensity in an entire wavelength band detected by a light receiver LR and a curing-light applying time.
Fig. 3 is a flowchart in which a cure end point monitored bonding process is outlined.
Figs. 4A, 4B, and 4C are explanation views for supportively explaining the process in Fig. 3.
Fig. 5 is a configuration view of a light-cure resin curing device 10.
Fig. 6 is a configuration view of a light-cure resin curing device 10 of another mode.
Fig. 7 is a configuration view of a light-cure resin curing device 10 of still another mode.
Fig. 8 is an explanation view for explaining another method.
Fig. 9 is a view showing a device configuration example of a type in which transmitted light through resin is detected as monitor light.
Fig. 10 is a view showing a device configuration example of a type in which reflected light from resin is detected as monitor light.
Fig. 11 is a graph showing a measurement result on first resin.
Fig.12 is a graph showing a measurement result on the first resin.
Fig. 13 is a graph showing a measurement result on the first resin.
Fig. 14 is a graph showing a measurement result on the first resin.
Fig. 15 is a graph showing a measurement result on second resin.
Fig. 16 is a graph showing a measurement result on the second resin.
Fig. 17 is a graph showing a measurement result on the second resin.
Fig. 18 is a graph showing a measurement result on the second resin.
Fig. 19 is a graph showing a measurement result on third resin.
Fig. 20 is a graph showing a measurement result on the third resin.
Fig. 21 is a graph showing a measurement result on the third resin.
Fig. 22 is a graph showing a measurement result on the third resin.
Fig. 23 is a graph showing a measurement result on the third resin.
Fig. 24 is a graph showing a measurement result on the third resin.
Fig.25 is a graph showing a measurement result on a prepared slide.
Fig. 26 is a graph showing a measurement result on the prepared slide.
Fig. 27 is a graph showing a measurement result on the prepared slide.

### Best Modes for Carrying Out the Invention

Hereinafter, a description will be made of a resin cure end point detection method, a cure end point detection device, an assembly, assembly manufacturing apparatus and method according to an embodiment. Note that same numerals will be used for like components and redundant descriptions will be omitted.

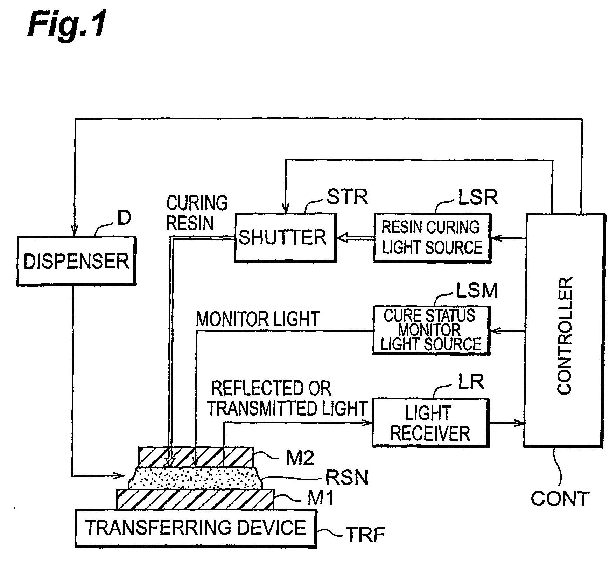

Fig. 1 is a block diagram of an assembly manufacturing apparatus including a resin cure end point detection device. Relevant resin RSN is interposed between first and second members M1 and M2 which are to be bonded together. An assembly of the embodiment is composed of the first and second members M1 andM2 and the resin RSN. The resin RsN is supplied between the members M1 and M2 from a dispenser D. The resin RSN is light-cure resin. When resin curing light (ultraviolet light, visible light, or the like; depending on the resin) is applied from a resin curing light source LSR to the resin RSN, a polymer material which is a main component of the resin RSN polymerizes and thus the resin RSN is cured.

When monitor light in a wavelength band different from the foregoing or weak monitor light in the same wavelength band as the foregoing is applied from a cure status monitor light source LSM to the resin RSN, a specific component of the monitor light is absorbed and so on and is attenuated within the resin RSN. Thereafter the monitor light enters a light receiver LR. Where the monitor light reflected by the resin RSN is referred to as a reflected light and where the monitor light transmitted through the resin RSN is referred to as a transmitted light, the light receiver LR detects any one of the reflected light and the transmitted light.

The light receiver LR has a configuration in which a photo diode or a photo diode array and a spectroscope are combined. The light receiver can detect a spectrum in a predetermined wavelength band in a received light. Light intensities at a plurality of wavelengths which are outputted from the light receiver LR are inputted to a controller CONT. The controller CONT controls a shutter (stopping means) STR for shutting out the curing light, a transferring device TRF for transferring an assembly composed of the members M1 and M2, and the dispenser D. In addition, based on the above described data inputted from the light receiver LR, the controller CONT judges cure end time of the resin RSN.

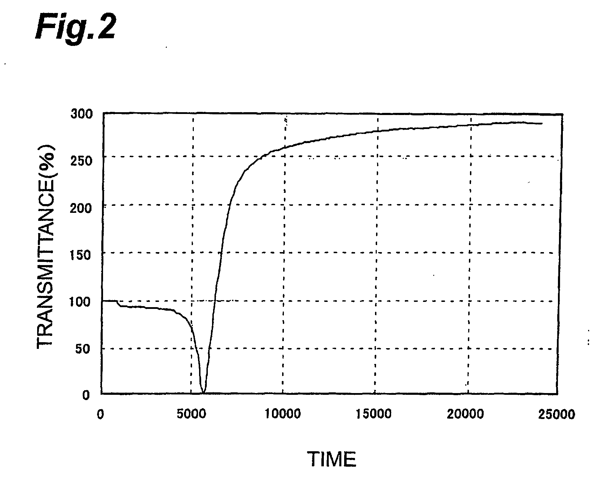

Fig. 2 is a graph showing a relationship between a light intensity in the entire wavelength band which is detected by the light receiver LR and an applying time of the curing light. The light intensity falls with the applying time once and thereafter increases. In this way, a temporal change of an intensity of transmitted light through resin RSN is significant while the resin RSN is being cured. As known heretofore, the intensity of transmitted light through resin RSN does not always change monotonically. It is conceivable that this is because not only a transmittance of resin RSN but also a refractive index thereof changes as cure of the resin RSN progresses. In other words, it is conceivable that the resin RSN functions like a kind of optical lens due to the cure, whereby a change of the transmittance to be measured is resultingly affected.

In a case where the curing light is transmitted through a member to be bonded, the light to be measured includes an influence of the member. Therefore, in a case where the cure of the resin RSN does not progress uniformly in full or in a case where the member to be bonded is changed, it is absolutely impossible to perform an accurate end point detection by a conventional judging method such as measurement of an absolute value of a transmittance. Although the transmittance changes depending on a vibration frequency of a polymerization reaction-related binding site of a polymer, simple measurement of this transmittance provides only a rough indication but does not provide an accurate cure end point detection.

The resin cure end point detection method according to the embodiment is a method including the steps of applying the monitor light from the light source LSM to the resin RSN, detecting the monitor light which is reflected by the resin RSN or which is transmitted through the resin RSN, and detecting a cure endpoint of the resin RSN. Here, based on an intensity I(0) of the monitor light at least at one specific wavelength which is detected before the cure of the resin RSN and based on an intensity I (t) of the monitor light at the specific wavelength which is thereafter detected, the cure of the resin RSN is judged to be completed.

When the intensity of the monitor light at least at the one specific wavelength which is detected before the cure of the resin RSN and the intensity of the monitor light at the specific wavelength which is thereafter detected are used, it is possible to perform an accurate cure end point judgment because these intensities are deeply related to the cure end point. It is preferred to apply the monitor light to the resin before curing the resin to detect the monitor light before the cure.

As described above, the intensities before and after the cure are deeply related to the cure end point. Accordingly, a correlation value R (ratio (=I(t)/I(0)) in this example) between the intensity I (0) of the monitor light at least at the one specific wavelength which is detected before the cure of the resin RSN and the intensity I (t) of the monitor light with the specific wavelength which is thereafter detected is determined, and then, in a case where a temporal change of the correlation value R comes to a stable state, the cure of the resin RSN is judged to be completed. This operation is performed by the controller CONT.

To explain the correlation value R in the example in detail, the correlation value R is a ratio between the intensities at the specific wavelength before and after the cure of the resin RSN. In the example, for a purpose of confirming the stable state, a time derivative of the correlation value R is determined, and thereafter, in a case where the determined time derivative becomes almost zero (=0 +/- a predetermined value (very small value)), the cure of the resin RSN is judged to be completed. Note that a ratio between light intensity time derivatives at the specific wavelength before and after the cure may be used as the correlation value. Note that "almost zero" means that, where a correlation value is R, a time derivative (ΔR per minute) of the correlation value is in a range between -3% of R and +3% of R inclusive.

When the cure of the resin RSN is judged to be completed, the controller CONT sends a control signal to the shutter STR and closes the shutter STR to stop applying the curing light to the resin RSN. Thereafter, the controller CONT moves the current assembly composed of the members M1 and M2 from a curing light applying position by the transferring device TRF and moves a next assembly to the curing light applying position by the transferring device TRF. As a matter of course, the current assembly has been moved to the curing light applying position after cure of resin in an assembly prior to the current assembly is completed.

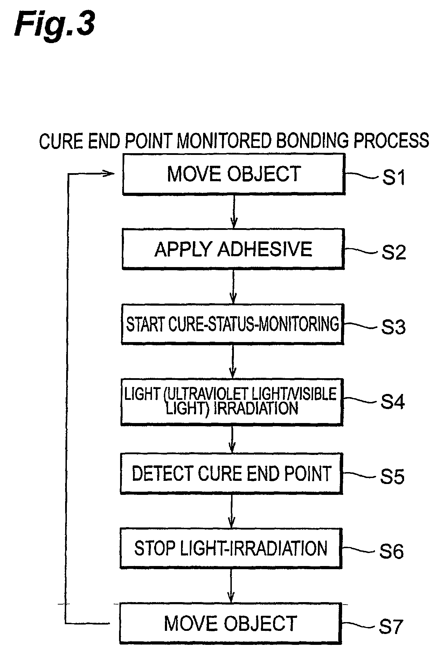

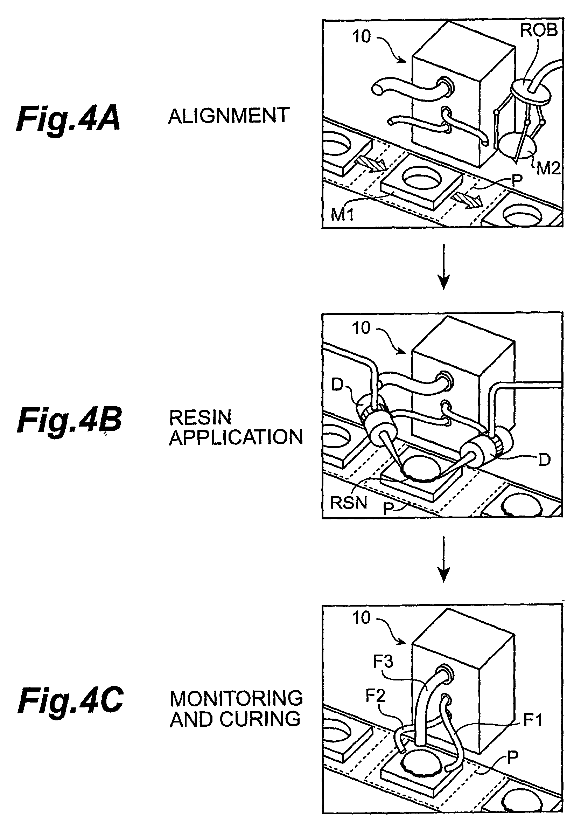

Fig. 3 is a flowchart in which a cure end point monitored bonding process is outlined. Figs. 4A, 4B, and 4C are explanation views for supportively explaining the cure end point monitored bonding process.

In the method, first, the assembly (object) is moved to the curing light applying position P (S1) (Fig. 4A) . The curing light applying position P is a position to which the curing light and the monitor light are capable of being applied from a light-cure resin curing device 10 having the resin cure end point detection device therein.

Next, the member M2, such as an objective lens, is located on the member M1, such as a lens holder, by a robot ROB, and then the resin RSN is applied and supplied to a gap between the members M1 and M2 from the dispenser D (S2) (Fig. 4B). A sequence of performing these locating step and resin supplying step may be reversed.

Thereafter, monitoring of resin cure status is started (S3) (Fig. 4C). The device 10 is provided with an optical fiber (light guide) F1 for guiding the monitor light, which is emitted from the monitor light source LSM, to the resin RSN; an optical fiber (light guide) F2 for guiding the monitor light from the resin RSN to the light receiver LR; and optical fibers (light guides) F3 for guiding the curing light, which is emitted from the resin curing light source LSR, to the resin RSN. Note that, in the example, it is assumed that each of the optical fibers F1 and F2 for monitoring is one optical fiber, and that the optical fibers F3 for curing are a plurality of optical fibers (optical fiber bundle).

When the monitoring of the resin cure status is started, the monitor light from the monitor light source LSM is applied to the resin RSN through the optical fiber F1, and the monitor light from the resin RSN enters the light receiver LR through the optical fiber F2. Therefore, a spectrum of the light entering the light receiver LR is being stored on the controller CONT at a predetermined sampling timing.

In a case where the resin RSN is acrylic adhesive, an ethylenic double bond (=CH2) has an absorption spectrum at 1617 nm, and this bond is cut when the resin RSN is polymerized. Accordingly, in a whole spectrum, light with a wavelength of 1617 nm is used as the light with the specific wavelength, and an intensity I(0) of the light with the wavelength of 1617 nm at monitoring-start time is measured. In this case, the intensity I falls gradually as the cure progresses, and a change of the intensity I stops when the cure is completed.

In a case where the resin RSN is epoxy adhesive, a single bond (CH-CH) and a CONH2 bond have absorption spectra at 1166 nm and 1429 nmn, respectively. Accordingly, in a whole spectrum, light with a wavelength of 1166 nm and light with a wavelength of 1429 nm are used as the light with the specific wavelength, and intensities I(0) of the light with the wavelength of 1166 nm and the light with the wavelength of 1429 nm at monitoring-start time are measured. In this case, the intensity I of the light with the wavelength of 1166 nm increases gradually as the cure progresses, and a change of the intensity I stops when the cure is completed. On the other hand, the intensity I of the light with the wavelength of 1429 nm falls gradually as the cure progresses, and a change of the intensity I stops when the cure is completed.

Thereafter, the curing light emitted from the resin curing light source LSR is applied to the resin RSN through the optical fibers F3, and thus the curing of the resin RSN is started (S4). While this state is being maintained, the monitor light enters the light receiver LR, and thus the spectrum of the monitor light is being stored on the controller CONT. The intensity at the specific wavelength is denoted by I (t) .

The controller CONT performs the above described judging process continuously. When the cure of the resin is judged to be completed (S5), the controller CONT closes the shutter SHR to stop applying the curing light to the resin RSN (S6). In a case where there is one specific wavelength, the cure end point of the resin is defined as time when a temporal change of a correlation value R (=I(t)/I (0)) at the specific wavelength disappears. In a case where there are two specific wavelengths, the cure end point of the resin is defined as time when temporal changes of correlation values R at both the specific wavelengths disappear, that is, time when time derivatives thereof become almost zero. In a case where there are three or more specific wavelengths, it is judged that time when changes of correlation values R at all the specific wavelengths disappear is the cure end point of the resin.

Finally, the assembly in which the cure of the resin is completed is moved from the curing light applying position P (S1), and then the next assembly is moved to the position P(57). Hereafter, the above described process is repeated.

In other words, the assembly manufacturing method in which the above described light-cure resin is used comprises the steps of: interposing the light-cure resin RSN between the members M1 and M2 of the assembly which includes at least the two members M1 and M2, transferring the assembly to the position P to which the resin curing light for curing the resin RSN is applied, and applying the resin curing light to the resin RSN; and applying the monitor light to the resin RSN before applying the resin curing light, detecting the monitor light which is reflected by the resin RSN or which is transmitted through the resin, detecting the cure end point of the resin RSN based on a result of the foregoing detecting, stopping application of the resin curing light to the resin RSN, and transferring the assembly from the position P.

In the above described process, the step of interposing the resin and the transferring of the assembly can be performed in random order. "Transferring an assembly" means that an assembly is resultingly transferred to the above described position.

According to the method, the monitor light is applied to the resin RSN before the resin curing light is applied, and the monitor light which is reflected by the resin RSN or which is transmitted through the resin is detected. Based on a result of this detection, the cure end point of the resin is detected. Thereafter, application of the resin curing light to the resin RSN is stopped, and the assembly is transferred from the curing light applying position P. Accordingly, it is possible to manufacture an assembly efficiently. An assembly in which resin is cured according to the manufacturing method can reduce manufacturing cost thereof.

As such an assembly, there are enumerated an optical pickup device and the like, which are described in Japanese Unexamined Patent Publication No. Hei10(1998)-39120, Japanese Unexamined Patent Publication No. Hei8(1996)-329508, Japanese Unexamined Patent Publication No. Hei9 (1997)-35312, Japanese Unexamined Patent Publication No.Hei4(1992)-344792, and Japanese Unexamined Patent Publication No. Hei5(1993)-45288.

Next, a description will be made of an internal configuration of the light-cure resin curing device 10.

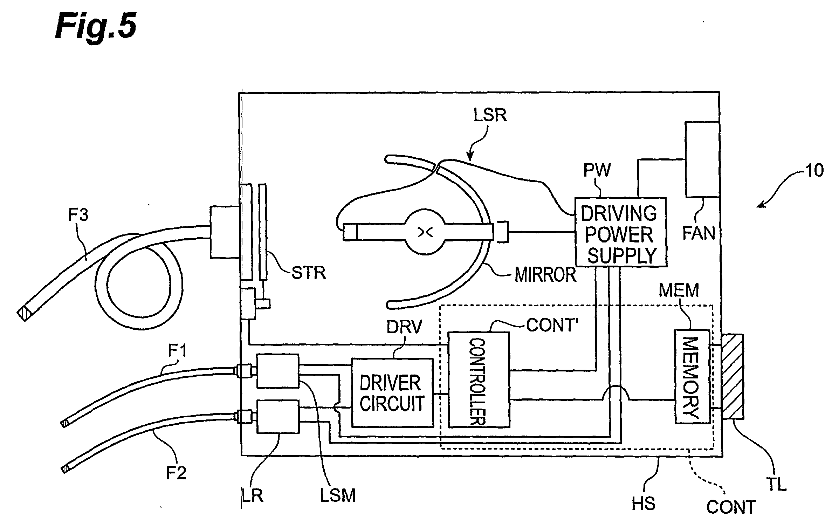

Fig. 5 is a configuration view of the light-cure resin curing device 10. The device 10 includes the curing light source LSR, the monitor light source LSM, and the light receiver LR, which are contained inside a hard shell HS; a driver circuit DRV for turning on the monitor light source LSM and for amplifying an output signal from the light receiver LR; an air cooling fan FAN for cooling an inside of the hard shell HS; the controller CONT (CONT', MEM); the shutter STR of which open and close are controlled by the controller CONT; a driving power supply PW for supplying power to the above described constituents; the optical fibers F1/ F2, and F3 which are exposed outside the hard shell HS; and an external input/output terminal TL for sending data to an external device and for receiving data from the external device.

The controller CONT is shown in such a manner that a memory MEM for storing a spectrum as described above is separated from a controller main body CONT'. The controller main body CONT' controls the driver circuit DRV, the shutter STR, and the memory MEM and performs the above described end point judgment. The controller CONT may have a control function for the dispenser D and the transferring device TRF which are shown in Fig. 1. Alternatively, this control function may be performed by a controller outside the device.

In the device 10, the curing light emitted from the curing light source LSR enters the optical fiber F3 which is optically coupled with the curing light source LSR, and is emitted from an end portion of the optical fiber F3. The light emitted from the monitor light source LSM enters the optical fiber F1 which is optically coupled with the monitor light source LSM, and is emitted from an end portion of the optical fiber F1. The monitor light from the resin RSN enters the light receiver LR which is optically coupled with the optical fiber F2.

As the curing light source LSR, it is possible to use a mercury xenon lamp, a mercury lamp, a metal halide lamp, an excimer lamp, a semiconductor laser diode, or the like which is provided with a reflecting mirror.

As the monitor light source LSM, it is possible to use a halogen lamp, a metal halide lamp, a light-emitting diode, a semiconductor laser diode, or the like.

As a method of curing the resin RSN, various methods are conceivable. Since the device performing the relevant method is further provided with the resin curing light source LSR emitting light with the wavelength for curing the resin to the resin RSN, the curing can be started by use of the light. In this case, if the light source LSM for emitting the monitor light is identical to the resin curing light source LSR, it is possible to simplify a configuration of the device.

As a light receiving element used in the light receiver LR, it is possible to use a semiconductor photo detector (such as an InGaAs photodiode array), a phototube, an electron-bombarded CCD, a photo multiplier tube, or the like.

Note that, for the light-cure resin curing device 10, another configuration may be adopted.

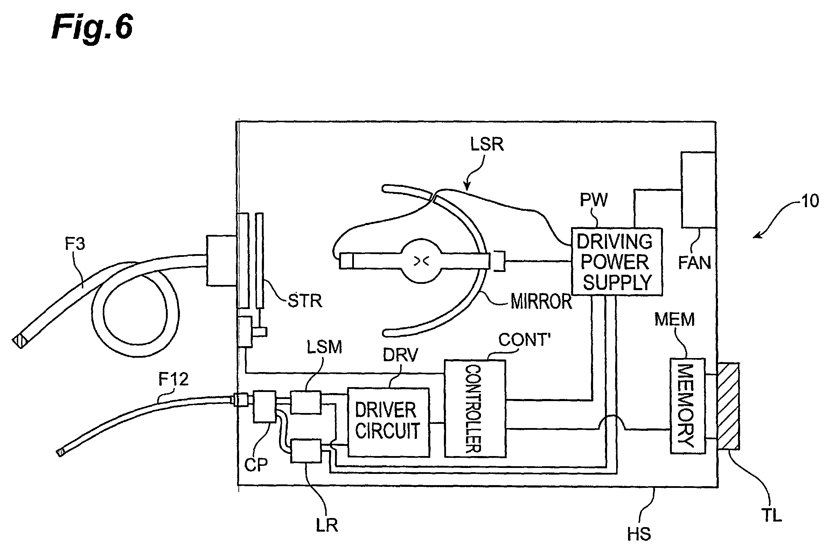

Fig. 6 is a configuration view of a light-cure resin curing device 10 of another mode. This light-cure resin curing device 10 is different from the light-cure resin curing device 10 shown in Fig. 5 in that a two-branched optical fiber coupler CP in which the light emitted from the monitor light source enters one input port thereof is used, in that the other input port thereof is coupled with the light receiver LR optically, and in that one optical fiber F12 is used for an output port of the optical fiber coupler CP instead of the optical fibers F1 and F2.

The monitor light emitted from the light source LSM is entered into the coupler CP and is emitted from the optical fiber 12. The monitor light having entered the optical fiber 12 from a direction of the resin RSN enters the light receiver LR through the coupler CP. Except for this, the configuration is the same as the configuration shown in Fig. 5.

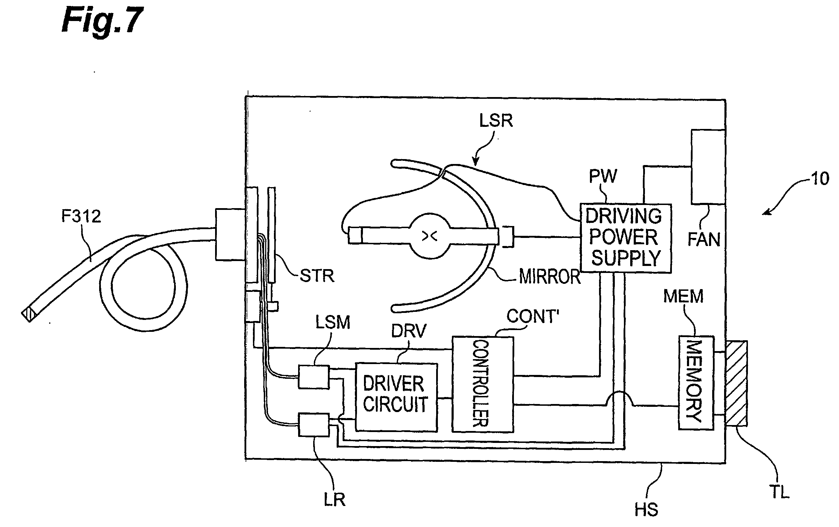

Fig. 7 is a configuraition view of a light-cure resin curing device 10 of still another mode. This light-cure resin curing device 10 is different from the light-cure resin curing device 10 shown in Fig. 5 in that an optical fiber bundle F312 is used instead of the optical fibers F1, F2, and F3, and in that one optical fiber thereof equivalent to the optical fiber F1 and one optical fiber thereof equivalent to the optical fiber F2 are respectively coupled with the monitor light source LSM and the light receiver LR optically.

The monitor light emitted from the light source LSM is emitted from the optical fiber F312, and the monitor light having entered the optical fiber F312 from the direction of the resin RSN enters the light receiver LR. Note that the curing light is also emitted toward the direction of the resin RSN through the optical fiber F312. Except for this, the configuration is the same as the configuration shown in Fig. 5.

Next, a description will be made of a method of interposing the resin RSN between the members M1 and M2, applying pressure, and curing the resin RSN.

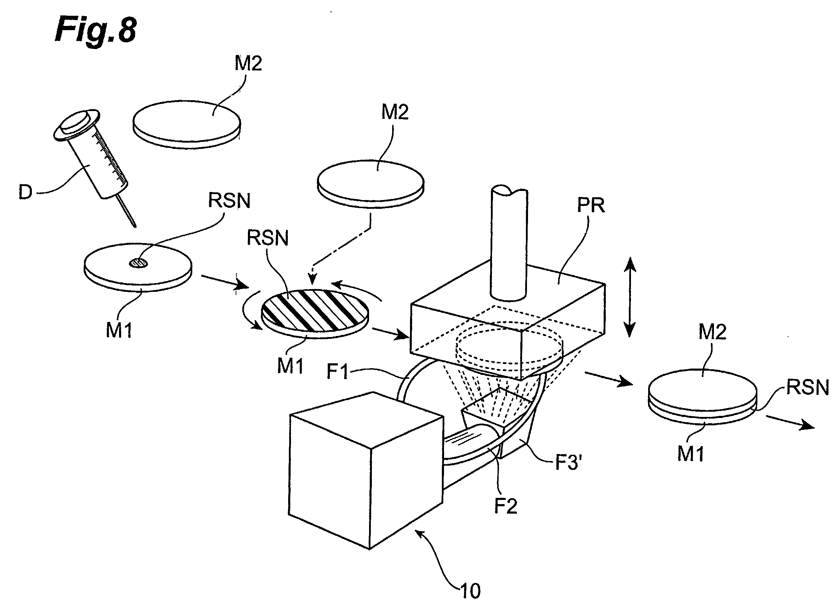

Fig. 8 is an explanation view for explaining such a method. In this method, first, the resin RSN is supplied from the dispenser D to a surface of the member M1 such as a glass plate. Next, the member M1 is rotated by a spinner, and thus the resin RSN is dispersed uniformly on the surface of the member M1. Furthermore, the member M2 is located on the member M1, and then this assembly is transferred to the curing light applying position to be pressed in a thickness direction of the members M1 and M2 by a pressing device PR. Thereafter, the light-cure resin curing device 10 performs the above described resin curing process and detects the cure end point of the resin RSN. The assembly in which the cure is completed is transferred from the curing light applying position. Note that, in the light-cure resin curing device 10 shown in the drawing, an optical system F3' composed of a mirror, a lens, and the like is used instead of the optical fiber F3 in Fig. 5.

As described above, according to the method of the embodiment, when the temporal change of the correlation value R comes to a stable state, the cure of the resin RSN is judged to be completed. Accordingly, it is possible to perform an accurate cure end point judgment. The resin cure end point detection device performing this method includes the light source LSM for emitting the above described monitor light, the light receiver LR for detecting the above described monitor light, and the controller (judging means) CONT for performing the above described judgment.

The manufacturing apparatus for performing the above described manufacturing method includes the resin curing light source LSR which applies the resin curing light for curing the light-cure resin RSN to the resin, the cure status monitor light source LSM for applying the monitor light to the resin RSN, the light receiver LR for detecting the monitor light which is reflected by the resin RSN or which is transmitted through the resin RSN, the shutter (stopping means) STR for stopping application of the resin curing light to the resin RSN, and the controller CONT for detecting the cure end point of the resin RSN based on the output of the light receiver LR and for controlling the shutter STR to stop the application of the resin curing light to the resin RSN.

Note that, though it is possible to use as the stopping means STR a power supply switch of the resin curing light source, the shutter interposed in a light path between the resin curing light source LSR and the resin RSN is adopted from a viewpoint of output stability.

Next, a description will be made of a specific example of a cure end point detection device adopting this configuration.

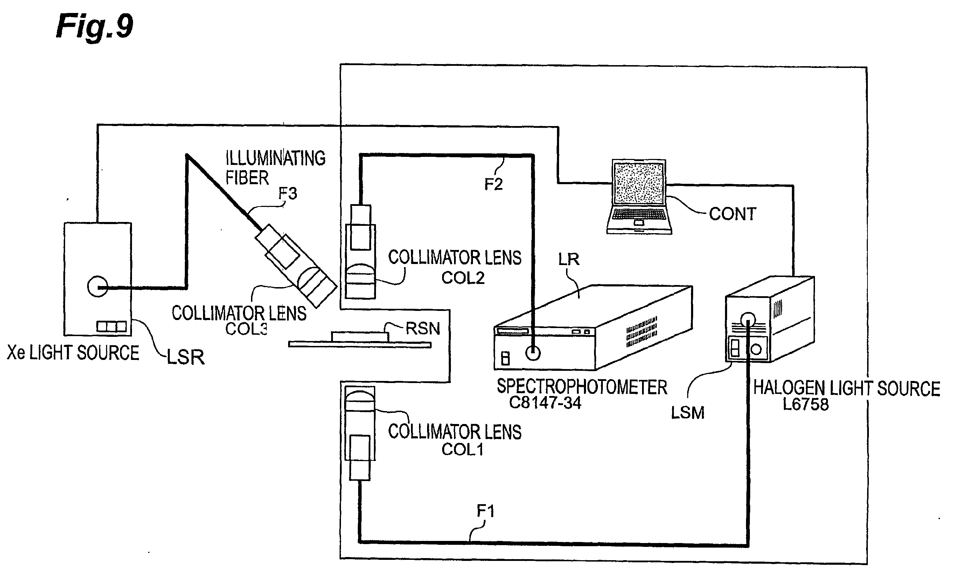

Fig. 9 is a view showing a device configuration example of a type in which the transmitted light through resin is detected as monitor light. In the example, a UV spot light source device (Hamamatsu Photonics K.K., L7212-01) is used as a resin curing light source LSR. Curing light emitted from the light source LSR is emitted through an optical fiber (Hamamatsu Photonics K.K., A2873) F3. The curing light from the optical fiber F3 is made into parallel light with a collimator lens (Hamamatsu Photonics K.K., E5147-06) COL3 which is attached to a tip portion of the fiber F3, and then the curing light is applied to the resin RSN.

As a monitor light source LSM, a halogen light source (Hamamatsu Photonics K.K., L6758) is used. The monitor light emitted from the light source LSM is emitted through an optical fiber F1. The monitor light from the optical fiber F1 is collimated into parallel light with a collimator lens COL1 which is attached to a tip portion of the fiber F1, and then the monitor light is applied to the resin RSN.

The monitor light transmitted through the resin RSN is condensed with a collimator lens COL2 which is attached to a tip portion of a fiber F2. The condensed monitor light enters an end surface of the optical fiber F2 and is transmitted through the optical fiber F2 to be entered into the light receiver LR. As a light receiver LR, a spectrophotometer (Hamamatsu Photonics K.K., C8147-34) can be used. An output of the light receiver LR is inputted to a controller CONT for performing data analysis. The controller CONT detects a cure end point of the resin as described above.

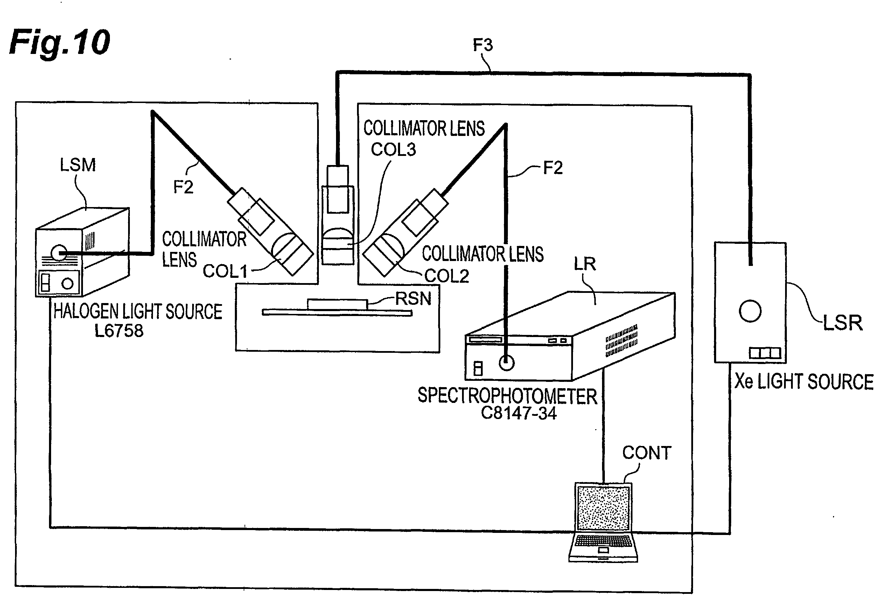

Fig. 10 is a view showing a device configuration example of a type in which reflected light from resin is detected as monitor light. This device is different from the device shown in Fig. 9 in that collimator lenses COL1 and COL2 are arranged at positions where the monitor light can be applied to the resin RSN and where the monitor light reflected by the resin RSN can be measured. Except for this, the configuration is the same as the configuration shown in Fig. 9. Note that an incident angle of curing light to the resin RSN can be set to zero degrees or an appropriate angle less than 90 degrees, for example, 45 degrees.

By using this device, a relationship between a curing light applying time and a monitor light transmission spectrum of resin was investigated in practice.

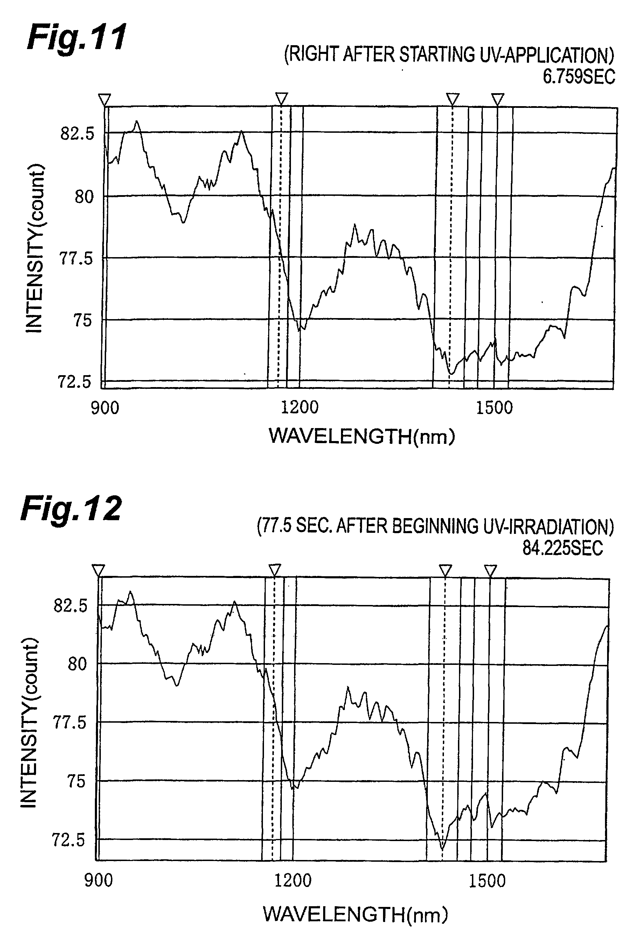

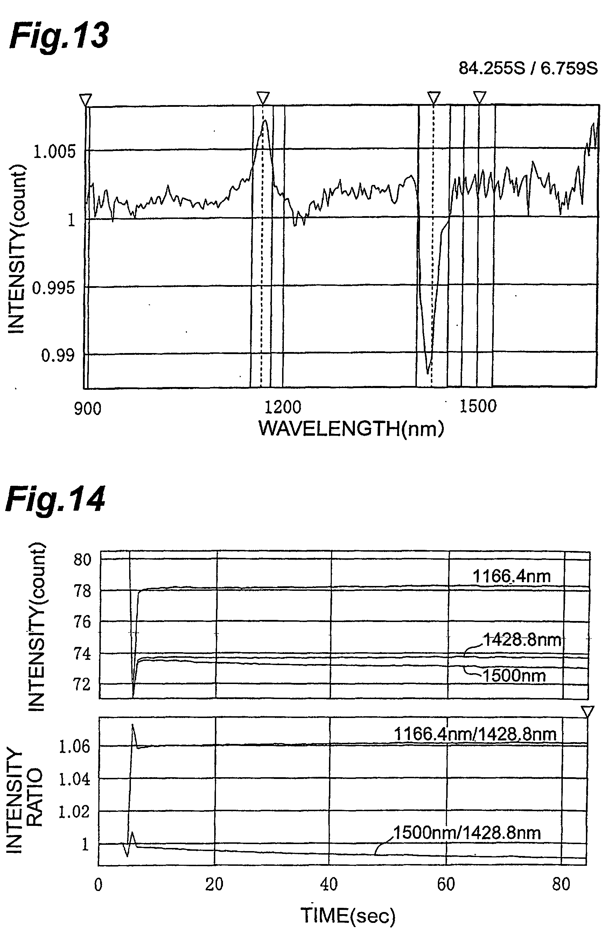

Figs. 11 to 14 show measurement results in a case where ultraviolet-cure epoxy resin (ThreeBond 3121) is used as first resin to be measured. Specific wavelengths are set to 1166.4 nm, 1428.8 nm, and 1500 nm in a whole obtained spectrum. After Δt (=6.759) seconds from a start of cure status monitoring (t=0), curing light (ultraviolet light) is applied to the resin (Fig.11) , and time (t=77.5 seconds) after 84.255 seconds from the start of the monitoring is judged to be cure end time (Fig. 12). Fig. 13 shows an intensity ratio between these, R=I (t=77.5) /I(0). After the cure end time, the intensity ratio R between these does not change. Note that, as shown in Fig. 14, in a case where only intensity ratios among the wavelengths are measured, the intensity ratios do not show significant changes. Note that, as to time T, monitoring-start time is defined as T=0. Specifically, the equation T=t+Δt is established.

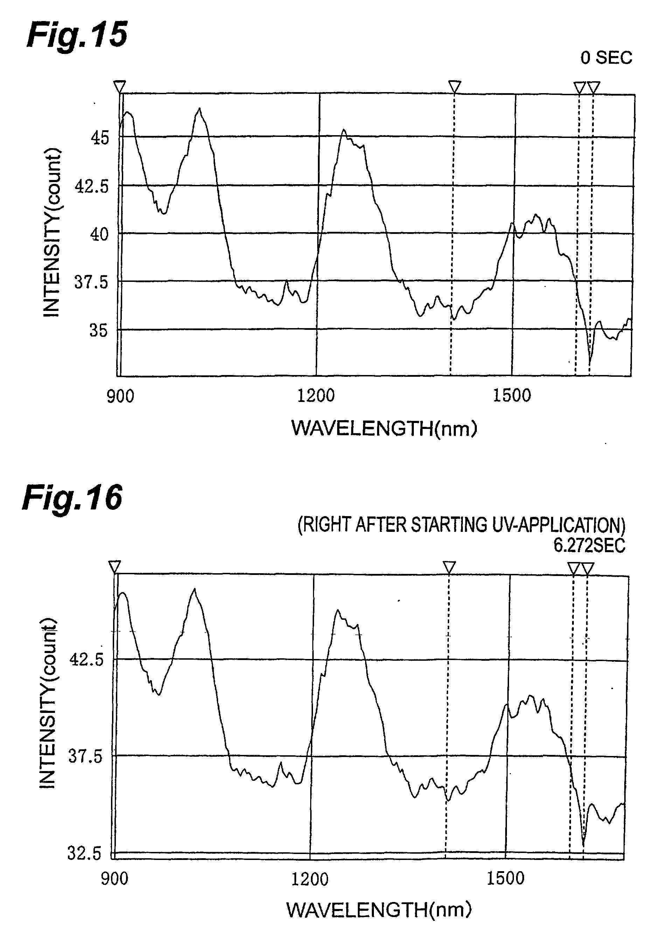

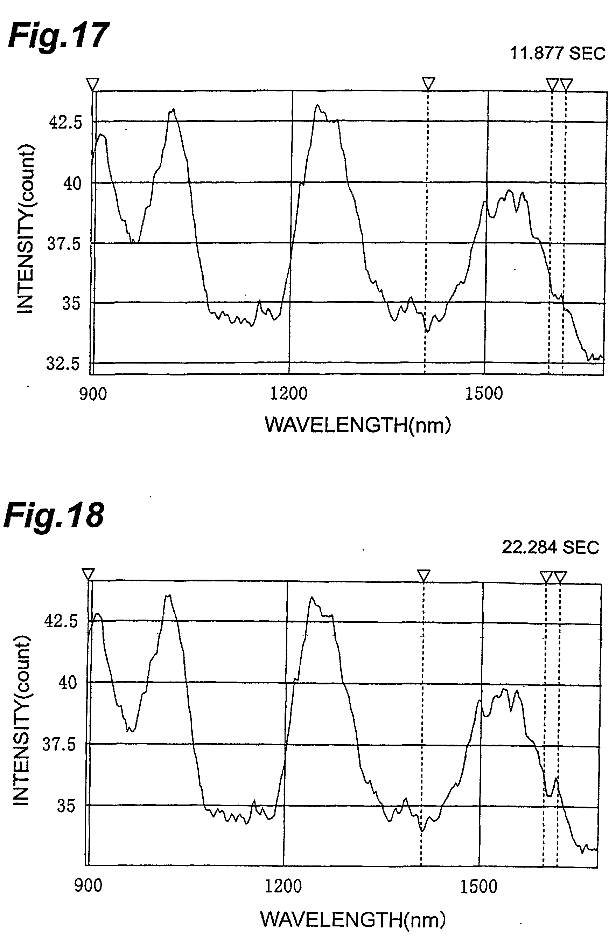

Measurement results in a case where modified acrylic resin (Multi-cure 625) is used as second resin to be measured are shown in Figs. 15 to 18. A cure time t of this resin is 10 seconds or less. Fig. 15 shows a spectrum in a case of T=0 second. Fig. 16 shows a spectrum in a case of T=6.272 seconds (curing-start time, t=0). Fig. 17 shows a spectrum in a case of T=11.877 seconds. Fig. 18 shows a spectrum in a case of T=22.284 seconds. It can be seen that, at specific wavelengths shown by triangle marks in the drawings, changes of intensities become almost zero after T=11.877 seconds.

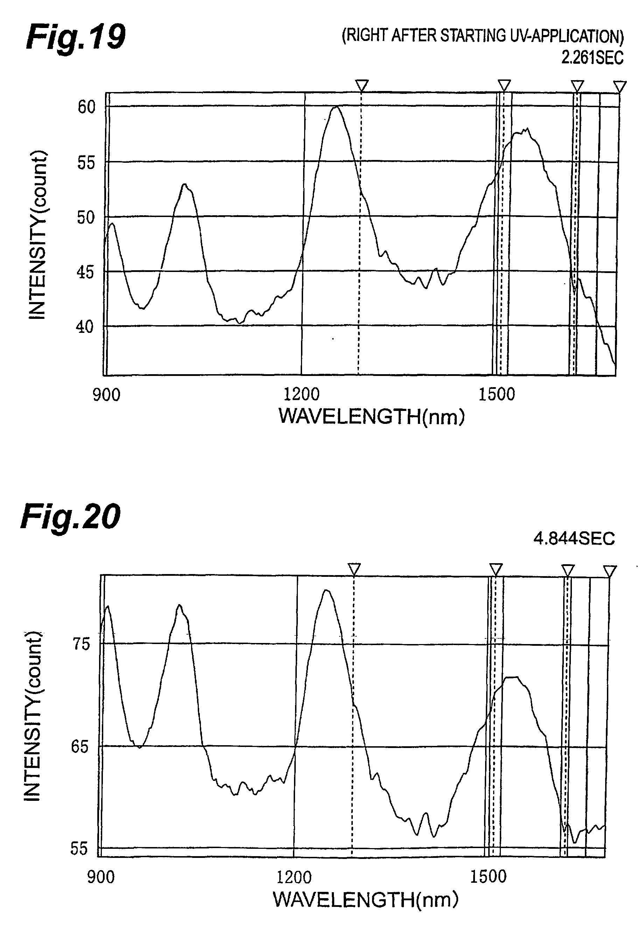

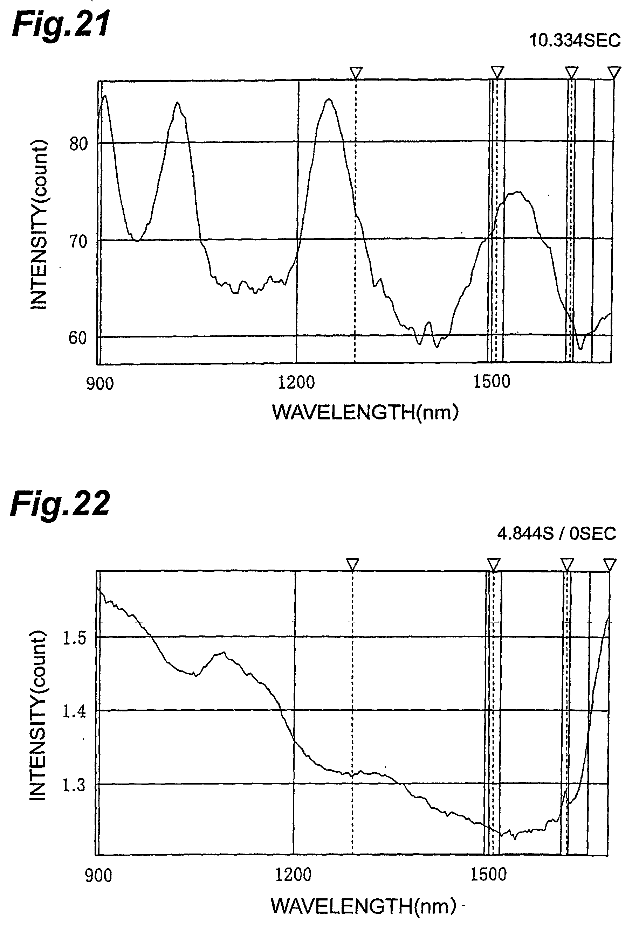

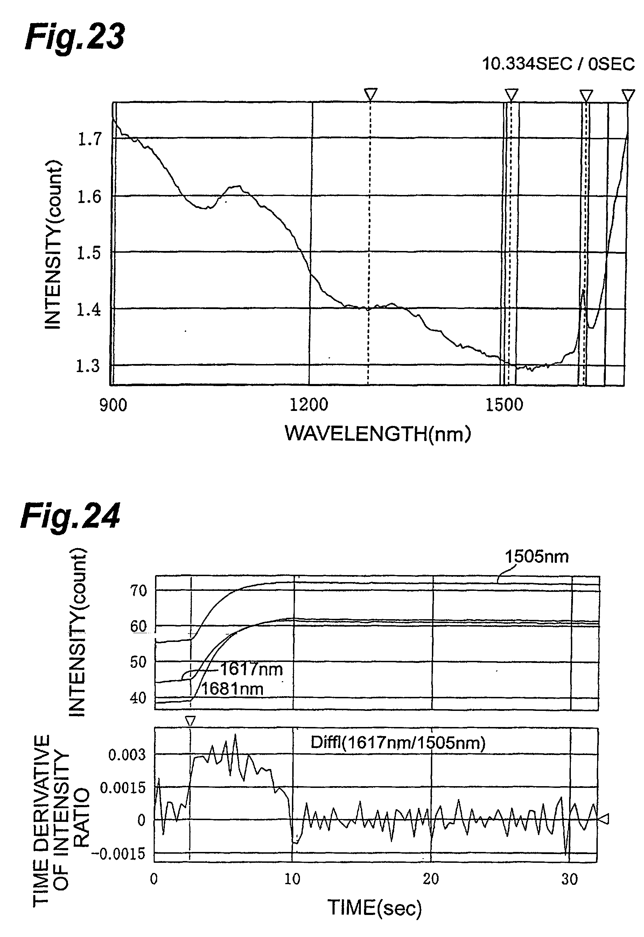

Figs. 19 to 24 show measurement results in a case where adhesive for an objective lens in a general optical pickup is used as third resin to be measured. Fig. 19 shows a spectrum in a case of T=2.261 seconds (t=0). Fig. 20 shows a spectrum in a case of T=4.844 seconds. Fig. 21 shows a spectrum in a case of T=10.344 seconds. Fig. 22 shows intensity ratios R=I(T=4.844)/I(T=0) at specific wavelengths which is shown by triangle marks in the drawing. Fig. 23 shows intensity ratios R=I (T=10.344) /I(T=0) at the specific wavelengths which is shown by triangle marks in the drawing. After T=10.334 seconds, there are almost no changes of the intensity ratios R.

Note that, as shown in Fig. 24, when an intensity ratio between the specific wavelengths (=1(1617 nm)/I(1505 nm)) is differentiated with respect to time, it can be seen that the change of the intensity ratio is significant and is not constant.

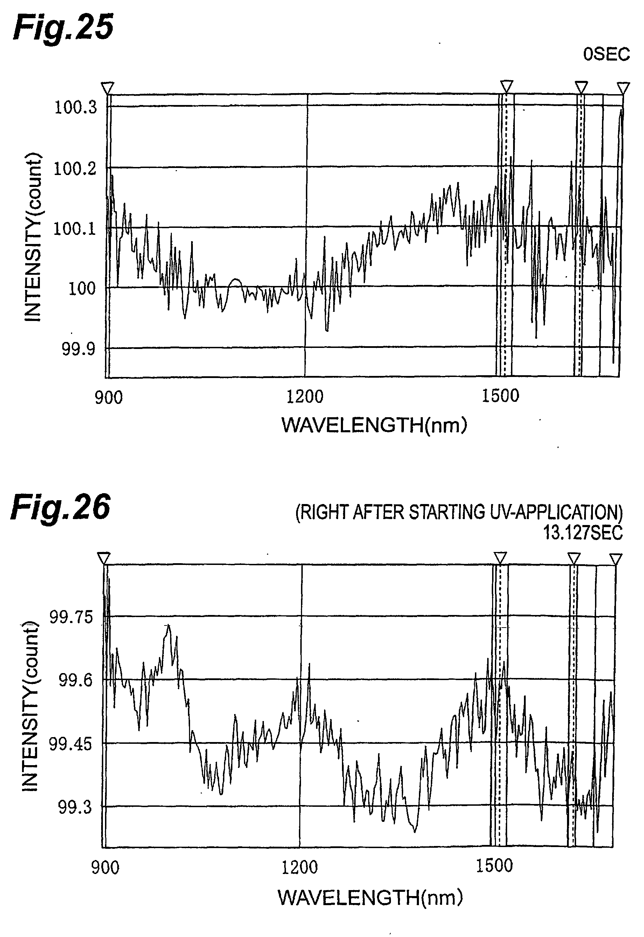

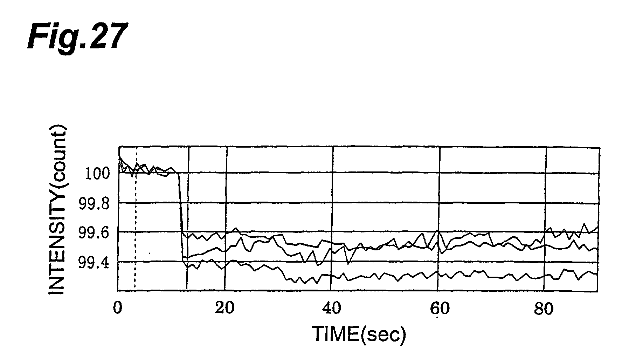

Figs. 25 to 27 show measurement results in a case where just a prepared slide is used as an object to be measured. Fig. 25 shows a spectrum in a case of T=0 second. Fig. 26 shows a spectrum in a case of T=13.127 seconds (curing-start time, t=0) . Fig. 27 shows temporal changes at three specific wavelengths shown by triangle marks in Figs. 25 and 26.

According to the above described resin cure end point detection device and the detection method thereof, it is possible to perform an accurate cure end point judgment. Moreover, according to the manufacturing method thereof, it is possible to manufacture an assembly efficiently, thus making it possible to reduce manufacturing cost thereof.

### Industrial Applicability

The present invention can be adapted to resin cure end point detection method and device, an assembly using light-cure resin, manufacturing apparatus and method thereof.

## Claims

1. A resin cure end point detection method of applying monitor light to resin, detecting any one of the monitor light reflected by the resin and the monitor light transmitted through the resin, and detecting a cure end point of the resin,
wherein, based on an intensity of the monitor light at least at one specific wavelength which is detected before cure of the resin and based on an intensity of the monitor light at the specific wavelength which is thereafter detected, the cure of the resin is judged to be completed.

2. The resin cure end point detection method according to claim 1,
wherein the monitor light before the cure is detected by applying the monitor light to the resin before curing the resin.

3. The resin cure end point detection method according to claim 1,
wherein a correlation value between the intensity of the monitor light at least at the one specific wavelength which is detected before the cure of the resin and the intensity of the monitor light at the specific wavelength which is thereafter detected is determined, and
the cure of the resin is judged to be completed when a temporal change of the correlation value comes to a stable state.

4. The resin cure end point detection method according to claim 1,
wherein the correlation value is a ratio at the specific wavelength between the intensities before and after the cure of the resin.

5. The resin cure end point detection method according to claim 2,
wherein a time derivative of the correlation value is determined, and
the cure of the resin is judged to be completed when the determined time derivative becomes almost zero.

6. A resin cure end point detection device comprising:
a light source for emitting the monitor light according to claim 1;
a light receiver for detecting the monitor light; and
judging means for performing the judgment.

7. The resin cure end point detection device according to claim 6, further comprising:
a resin curing light source which emits light with a wavelength for curing the resin to the resin.

8. The resin cure end point detection device according to claim 7,
wherein the light source for emitting the monitor light is identical to the resin curing light source.

9. A method of manufacturing an assembly in which light-cure resin is used, comprising the steps of:
interposing light-cure resin between members of an assembly which includes at lease two members, transferring the assembly to a position to which resin curing light for curing the resin is applied, and applying the resin curing light to the resin; and
applying monitor light to the resin before applying the resin curing light, detecting any one of the monitor light reflected by the resin and the monitor light transmitted through the resin, detecting a cure end point of the resin based on a result of the foregoing detecting, stopping application of the resin curing light to the resin, and transferring the assembly from the position.

10. An assembly,
wherein resin is cured by the method according to claim 9.

11. An apparatus for manufacturing an assembly in which light-cure resin is used, comprising:
a resin curing light source which applies resin curing light for curing light-cure resin to the resin;
a cure status monitor light source for applying monitor light to the resin;
a light receiver for detecting any one of the monitor light reflected by the resin and the monitor light transmitted through the resin;
stopping means for stopping application of the resin curing light to the resin; and
a controller for detecting a cure end point of the resin based on an output of the light receiver and for controlling the stopping means to stop the application of the resin curing light to the resin.

12. The apparatus for manufacturing an assembly in which light-cure resin is used according to claim 11,
wherein the stopping means is a shutter interposed in a light path between the resin curing light source and the resin.
